(19) Europäisches Patentamt
European Patent Office
Office européen des brevets

(11) EP 2 824 599 A1

(12) **EUROPÄISCHE PATENTANMELDUNG**

(43) Veröffentlichungstag:
14.01.2015 Patentblatt 2015/03

(51) Int Cl.:
*G06F 19/00* (2011.01)  *A61B 19/00* (2006.01)
*G06F 17/50* (2006.01)

(21) Anmeldenummer: 13003481.2

(22) Anmeldetag: **10.07.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **ISS Integrated Scientific**
**2562 Port (CH)**

(72) Erfinder:
• **Studer, Harald P.**
**3097 Liebefeld (CH)**

• **Riedwyl, Hansjörg**
**2503 Biel (CH)**
• **Büchler, Philippe**
**1407 Gossens (CH)**
• **Roberts, Cynthia J.**
**Columbus, OH 43220 (US)**

(74) Vertreter: **Rentsch Partner AG**
**Rechtsanwälte und Patentanwälte**
**Fraumünsterstrasse 9**
**Postfach 2441**
**8022 Zürich (CH)**

(54) **Vorrichtung und Verfahren zur Modellierung einer Hornhaut**

(57) Zur Modellierung einer Hornhaut für die Simulation von Gewebeschnitten in der Hornhaut, wird ein patientenspezifisches Finite Elemente Modell der Hornhaut erzeugt (S2). Im Finite Elemente Modell wird eine erste Gruppe von Gewebefasern, mit parallel zur Oberfläche der Hornhaut verlaufenden Hauptfasern, gemäss einer ersten Verteilungsfunktion verteilt (S22). Im Finite Elemente Modell wird überdies eine zweite Gruppe von Gewebefasern, mit inklinierten, nicht parallel zur Oberfläche der Hornhaut verlaufenden Crosslink-Fasern, gemäss einer zweiten Verteilungsfunktion verteilt (S23). Dabei verteilt (S23) die zweite Verteilungsfunktion die Crosslink-Fasern mit einer nicht uniformen Gewichtungsfunktion über die Tiefe der Hornhaut, von der äusseren Oberfläche der Hornhaut zur inneren Oberfläche der Hornhaut.

Fig. 4

EP 2 824 599 A1

**Beschreibung**

Technisches Gebiet

Die vorliegende Erfindung betrifft eine Vorrichtung und ein Verfahren zur Modellierung einer Hornhaut. Die vorliegende Erfindung betrifft insbesondere eine computerisierte Vorrichtung und ein computerimplementiertes Verfahren zur Modellierung einer Hornhaut für die Simulation von Gewebeschnitten in der Hornhaut, wobei die computerisierte Vorrichtung einen Prozessor umfasst, der programmiert ist, ein patientenspezifisches Finite Elemente Modell der Hornhaut zu erzeugen.

Stand der Technik

Zahlreiche Fehlsichtigkeiten wie Myopie (Kurzsichtigkeit), Hyperopie (Weitsichtigkeit) oder Astigmatismus (Stabsichtigkeit) werden heute mit chirurgischen Eingriffen am Auge korrigiert. Die refraktive Korrektur des Auges erfolgt dabei vorwiegend mittels ophthalmologischen Lasersystemen, welche das Augengewebe, insbesondere die Hornhaut (Cornea), schneiden und/oder abtragen, um die optische Brechkraft des Auges möglichst gut einem gewünschten Wert anzunähern. Gewebeschnitte können mittels Laserpulsen zwar äusserst präzise ausgeführt werden, viel genauer als vergleichsweise manuell mit einem Skalpell, doch die Veränderung der Gewebeform, die sich bei Gewebeschnitten aufgrund des intraokularen Drucks ergibt, macht die Planung von Schnitten und die Vorhersage von Schnittresultaten dennoch sehr schwierig.

Zur Verbesserung der ophthalmologischen refraktiven Korrektur beschreiben WO 02/07660, WO 94/18636, US 2009/318907 und US 2009/187386 die Modellierung des Auges mit der finiten Elemente Analyse. Dabei kann das Finite Elemente Modell des Auges für die Simulation von Gewebeschnitten verwendet werden. WO 02/07660 beschreibt die Einführung eines Schichtenmodells der Hornhaut, mit mehreren Schichten unterschiedlicher Härte. US 2009/318907 und US 2009/187386 beschreiben ein allgemeines achsensymmetrisches Modell, das auf einer nichtlinear elastischen, leicht komprimierbaren und transversal isotropischen Formulierung basiert. US 2009/187386 beschreibt überdies eine Anbindung der Hornhaut an die Sklera mit einer Vorprogrammierung von peripheren Elementen zur Abbildung einer festen Anbindung der Hornhaut an die Sklera (Limbus), was bei Schnitten, die über die Hornhaut hinaus in die angrenzende Sklera verlaufen, nicht ausreichend sein kann. Die bekannten Lösungen liefern überdies keine Hinweise wie der stromale Schwellungsdruck in die Modellierung einfliesst.

In den letzten Jahren wurde die Inhomogenität der Hornhaut über ihre Tiefe gründlich untersucht, insbesondere in:

Quantock AJ, Boote C, Yount RD, Hayes S, Tanioka H, Kawasaki S, Ohta N, Iida T, Yagi N, Kinoshita S, Meek KM, (2007); "Small-angle fibre diffraction studies of corneal matrix structure: a depth-profiled investigation of the human eye-bank cornea"; Journal of Applied Crystallography, 40: 335-340;

Palka BP, Tanioka H, Sotozono C, Yagi N, Boote C, Young RD, Meek KM, Quantock AJ, (2008); "Reduced collagen interfibrillar spacing in macular corneal dystrophy occurs predominantly in deep stromal layers"; Investigative Ophthalmology & Visual Science, 49: E-Abstract 3926;

Kamma-Lorger CS, Boote C, Young RD, Hayes S, Quantock AJ, Meek KM, (2008); "Depth profile study of molecular collagen structure in normal human cornea; Acta Ophthalmologica", 86: 243;

Meek KM, Boote C, (2009); "The use of X-ray scattering techniques to quantify the orientation and distribution of collagen in the corneal stroma"; Progress in Retinal and Eye Research, 28: 369-392;

Kamma-Lorger CS, Boote C, Hayes S, Moger J, Burghammer M, Knupp C, Quantock AJ, Sorensen T, Di Cola E, White N, Young RD, Meek KM, (2010); "Collagen and mature elastic fibre organization as a function of depth in the human cornea and limbus"; Journal of Structural Biology, 169: 424-430; und

Petsche SJ, Chernayak D, Martiz J, Levenston ME, Pinsky PM, (2012); "Depth-Dependent Transverse Shear Properties of the Human Corneal Stroma"; Investigative Ophthalmology & Visual Science, 53(2): 873-80.

Dabei wurde erkannt, dass das Gewebe über seine Tiefe, also von vorne/aussen (anterior) nach hinten/innen (posterior), immer schwächer wird und insbesondere die Schersteifigkeit deutlich abnimmt. Die Begründung wurde darin gefunden, dass es in den vorderen/äusseren Schichten viel inklinierte, also nicht parallel zur Hornhautoberfläche verlaufende Kollagenfasern gibt. Inklinierte Fasern, oder sogenannte Crosslink-Fasern, geben dem Gewebe die Schersteifigkeit. Dem gegenüber stehen die ordentlich aufeinander geschichteten, parallel zur Oberfläche verlaufenden Fasern in den tiefen (hinteren/inneren) Schichten.

Verschiedene mathematische Materialdefinitionen zur Simulierung der biomechanischen Eigenschaften des Hornhautgewebes wurden in der letzten Dekade erarbeitet und publiziert, insbesondere in:

Bryant M, McDonnell P, (1996); "Constitutive laws for biomechanical modelling of refractive surgery". Journal of Biomechanical Engineering, 118(4): 473-481;

Pinsky PM, Van der Heide D, Chernyak D, (2005); "Computational modelling of mechanical anisotropy in the cornea and sclera"; Journal of Cataract and Refractive Surgery, 31(1): 136-145;

Alastrue V, Calvo B, Pena E, Doblare M, (2006); "Biomechanical Modelling of Refractive Surgery; Journal of Biomechanical Engineering", 128: 150;

Lanchares E, Calvo B, Cristobal J, Doblare M, (2008); "Finite element simulation of arcuates for astigmatism correction"; Journal of Biomechanics, 41: 797-805;

Pandolfi A, Manganiello F, (2006); "A model for the human cornea: constitutive formulation and numerical analysis"; Biomechanics and Modelling in Mechanobiology, 5(4): 237-246;

Pandolfi A, Holzapfel G, (2008); "Three-dimensional modelling and computational analysis of the human cornea considering distributed collagen fibril orientations"; Journal of Biomechanical Engineering, 130(6); und

Pandolfi A, Fotia G, Manganiello F, (2009); "Finite element simulation of laser refractive corneal surgery"; Engineering with Computers, 25: 15-24.

Mathematische Materialdefinitionen zur Simulierung der biomechanischen Eigenschaften des Hornhautgewebes, in denen die inklinierten Fasern mitberücksichtigt werden, finden sich dabei einzig in den folgenden Schriften:

Studer HP, Larrea X, Riedwyl H, Büchler P, (2010); "Biomechanical model of human cornea based on stromal microstructure"; Journal of Biomechanics, 43: 836-842; und Petsche SJ, Pinsky PM, (2013); "The role of 3-D collagen organization in stromal elasticity: a model based on X-ray diffraction data and second harmonic-generated images"; Biomechanics and Modelling in Mechanobiology.

Dabei definieren Petsche SJ, Pinsky PM, in der letztgenannten Schrift eine dreidimensionale Verteilungsfunktion (alle Richtungen im sphärischen Koordinatensystem), welche gleichzeitig die Gewichtung der Haupt-, wie auch der inklinierten Fasern modelliert. Mittels dieser Gewichtung beschreiben die Autoren die Menge an Kollagenfasern die in einer bestimmten Richtung verlaufen. Die Fasern selber werden als Materialanisotropie im Kontinuum beschrieben.

Darstellung der Erfindung

Es ist eine Aufgabe der vorliegenden Erfindung eine Vorrichtung und ein Verfahren zur Modellierung einer Hornhaut (Cornea) vorzuschlagen, welche die Simulation von Gewebeschnitten in der Hornhaut ermöglichen. Es ist insbesondere eine Aufgabe der vorliegenden Erfindung eine Vorrichtung und ein Verfahren zur Modellierung einer Hornhaut vorzuschlagen, welche zumindest einige Nachteile der bekannten Systeme nicht aufweisen.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine computerisierte Vorrichtung zur Modellierung einer Hornhaut für die Simulation von Gewebeschnitten in der Hornhaut umfasst einen Prozessor, der programmiert ist, ein patientenspezifisches Finite Elemente Modell der Hornhaut zu erzeugen.

Die oben genannten Ziele werden durch die vorliegende Erfindung insbesondere dadurch erreicht, dass der Prozessor der computerisierten Vorrichtung überdies programmiert ist, eine erste Gruppe von Gewebefasern, mit parallel zur Oberfläche der Hornhaut verlaufenden Hauptfasern, gemäss einer ersten Verteilungsfunktion im Finite Elemente Modell zu verteilen, und eine zweite Gruppe von Gewebefasern, mit inklinierten, nicht parallel zur Oberfläche der Hornhaut verlaufenden Crosslink-Fasern, gemäss einer zweiten Verteilungsfunktion im Finite Elemente Modell zu verteilen, wobei die zweite Verteilungsfunktion die Crosslink-Fasern mit einer nicht uniformen Gewichtungsfunktion über die Tiefe der Hornhaut, von der äusseren Oberfläche der Hornhaut zur inneren Oberfläche der Hornhaut, verteilt.

In einer Ausführungsvariante ist der Prozessor programmiert, die Crosslink-Fasern mit einer abnehmenden Gewichtungsfunktion über die Tiefe der Hornhaut zu verteilen.

In einer Ausführungsvariante ist der Prozessor programmiert, im Finite Elemente Modell Permeabilitätswerte für Flüssigkeitsaustausch im Hornhautgewebe, einzuführen, welche von der Tiefe der Hornhaut abhängig sind.

In einer Ausführungsvariante ist der Prozessor programmiert, im Finite Elemente Modell Permeabilitätswerte für Flüssigkeitsaustausch im Hornhautgewebe, einzuführen, welche von der Gewichtungsfunktion abhängig sind, mit der die Crosslink-Fasern über die Tiefe der Hornhaut verteilt sind.

In einer Ausführungsvariante ist der Prozessor programmiert, einen Übergang vom patientenspezifischen Finite Elemente Modell der Hornhaut auf ein populationsbasiertes Skleramodell zu erzeugen, und die Crosslink-Fasern im Übergang mit einer abnehmenden Gewichtungsfunktion über die Tiefe des Übergangs zu verteilen, von einer äusseren Übergangsoberfläche, die sich von der äusseren Oberfläche der Hornhaut zur äusseren Oberfläche der Sklera erstreckt, zu einer inneren Übergangsoberfläche, die sich von der inneren Oberfläche der Hornhaut zur inneren Oberfläche der Sklera erstreckt.

In einer Ausführungsvariante ist der Prozessor programmiert, die Crosslink-Fasern im Finite Elemente Modell auf mehrere parallel zur äusseren Oberfläche der Hornhaut verlaufenden Schichten zu verteilen, und die Crosslink-Fasern in den Schichten jeweils mit einer zweidimensionalen Verteilungsfunktion zu verteilen.

In einer Ausführungsvariante ist der Prozessor programmiert, im Finite Elemente Modell unterschiedliche Materialeigenschaften für die erste Gruppe von Gewebefasern und die zweite Gruppe von Gewebefasern zu speichern.

In einer Ausführungsvariante ist der Prozessor programmiert, die Hauptfasern im Finite Elemente Modell gleichmässig auf mehrere parallel zur äusseren Oberfläche der Hornhaut verlaufende Flächen oder Schichten zu verteilen und auf den Flächen respektive in den Schichten jeweils mit einer zweidimensionalen Verteilungsfunktion zu verteilen.

Neben der computerisierten Vorrichtung zur Modellierung einer Hornhaut betrifft die vorliegende Erfindung überdies ein computerimplementiertes Verfahren zur Modellierung einer Hornhaut für die Simulation von Gewebeschnitten in der Hornhaut, sowie ein Computerprogrammprodukt, das ein nicht-transientes computerlesbares Medium mit darauf gespeichertem Computerprogrammcode umfasst, welcher eingerichtet ist, einen Prozessor derart zu steuern, dass der Prozessor das Verfahren zur Modellierung der Hornhaut ausführt. Das computerimplementierte Verfahren umfasst die Ausführung folgender Schritte durch den Prozessor: Erzeugen eines patientenspezifischen Finite Elemente Modells der Hornhaut; Verteilen im Finite Elemente Modell einer ersten Gruppe von Gewebefasern, mit parallel zur Oberfläche der Hornhaut verlaufenden Hauptfasern, gemäss einer ersten Verteilungsfunktion; und Verteilen im Finite Elemente Modell einer zweiten Gruppe von Gewebefasern, mit inklinierten, nicht parallel zur Oberfläche der Hornhaut verlaufenden Crosslink-Fasern, gemäss einer zweiten Verteilungsfunktion, wobei die zweite Verteilungsfunktion die Crosslink-Fasern mit einer nicht uniformen Gewichtungsfunktion über die Tiefe der Hornhaut, von der äusseren Oberfläche der Hornhaut zur inneren Oberfläche der Hornhaut, verteilt.

Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:

Figur 1:   zeigt ein Blockdiagramm einer computerisierten Vorrichtung zur Modellierung einer Hornhaut für die Simulation von Gewebeschnitten in der Hornhaut.

Figur 2:   zeigt ein Flussdiagramm mit einer beispielhaften Sequenz von Schritten eines Verfahrens zur Modellierung einer Hornhaut für die Simulation von Gewebeschnitten in der Hornhaut.

Figur 3:   zeigt ein Flussdiagramm mit einer beispielhaften Sequenz von Schritten zum Laden von Augendaten.

Figur 4:   zeigt ein Flussdiagramm mit einer beispielhaften Sequenz von Schritten zur Erzeugung eines Finite Elemente Modells einer Hornhaut für die Simulation von Gewebeschnitten in der Hornhaut.

Figur 5:   zeigt schematisch im Querschnitt eines Hornhautsegments die Verteilung von parallel zur Oberfläche der Hornhaut verlaufenden Hauptfasern über die Tiefe der Hornhaut (Tiefenverteilung) und eine entsprechende Verteilungsrespektive Gewichtungsfunktion.

Figur 6:   zeigt schematisch im Querschnitt eines Hornhautsegments die Verteilung von inklinierten, nicht parallel zur Oberfläche der Hornhaut verlaufenden Crosslink-Fasern über die Tiefe der Hornhaut (Tiefenverteilung) und eine entsprechende Verteilungs- respektive Gewichtungsfunktion.

Figur 7: zeigt schematisch im Querschnitt einen Übergang eines Hornhautsegments zu einem angrenzen Sklerasegment und die Verteilung von inklinierten, nicht parallel zur Oberfläche der Hornhaut verlaufenden Crosslink-Fasern über die Tiefe der Hornhaut und des Übergangs.

Figur 8: zeigt eine zweidimensionale Verteilungsfunktion für parallel zur Oberfläche der Hornhaut verlaufende Hauptfasern und inklinierte, nicht parallel zur Oberfläche der Hornhaut verlaufende Crosslink-Fasern.

Wege zur Ausführung der Erfindung

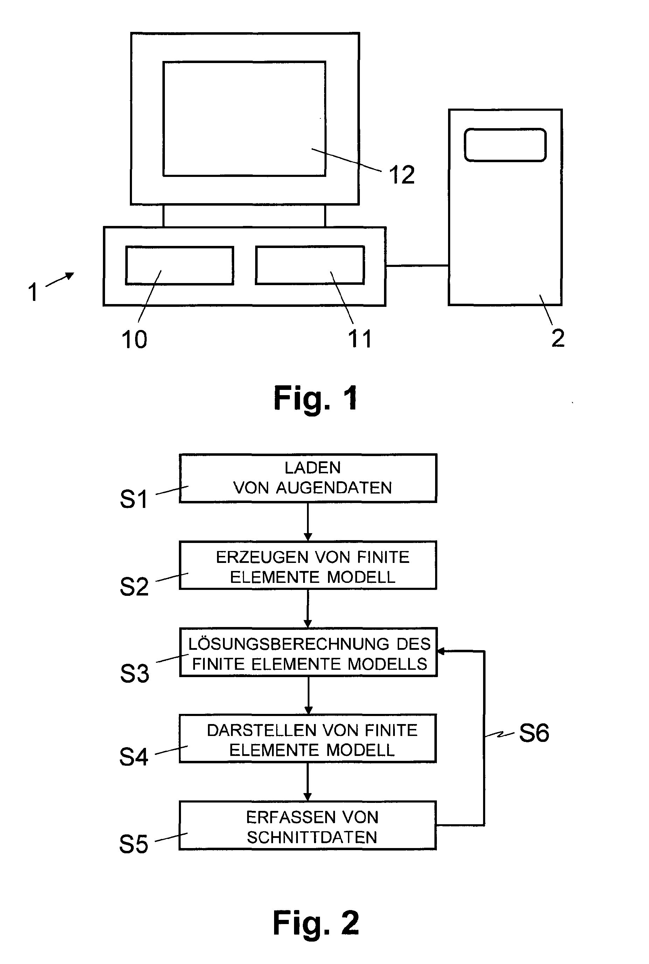

In der Figur 1 bezieht sich das Bezugszeichen 1 auf eine computerisierte Vorrichtung zur Modellierung einer Hornhaut für die Simulation von Gewebeschnitten in der Hornhaut. Die Vorrichtung 1 umfasst einen betriebsfähigen Computer mit mindestens einem Prozessor 10 und einem mit dem Prozessor 10 verbundenen Speicher 11 zur Speicherung von Daten (Datenspeicher) und Computerprogrammcode (Programmspeicher). In der Ausführung gemäss Figur 1 umfasst die Vorrichtung 1 überdies eine Anzeige 12, beispielsweise einen LED oder LCD Bildschirm. Wie in der Figur 1 schematisch dargestellt ist, ist die Vorrichtung 1 in einer Variante mit einer zur Vorrichtung 1 externen computerisierten Datenquelle 2 verbunden, beispielsweise über eine drahtgebundene oder drahtlose Kommunikationsschnittstelle. Die Datenquelle 2 umfasst einen oder mehrere Prozessoren und ist eingerichtet Augendaten, einschliesslich patientenspezifische Hornhautdaten und/oder populationsbasierte Skleradaten, an die Vorrichtung 1 zu übermitteln, wie später detaillierter beschrieben wird. Abhängig von der Ausführungsvariante umfasst die Datenquelle 2 eine ophthalmologische Messvorrichtung zur Erfassung der patientenspezifische Hornhautdaten von einem Patienten und/oder eine ophthalmologische Datenbank mit populationsbasierten Skleradaten.

Der Prozessor 10 ist eingerichtet respektive programmiert ein Verfahren zur Modellierung einer Hornhaut für die Simulation von Gewebeschnitten in der Hornhaut auszuführen. Die Vorrichtung 1 umfasst dazu ein Computerprogrammprodukt mit einem computerlesbaren Medium, das fest oder entfernbar mit dem Prozessor 10 verbunden ist und darauf gespeicherten Computerprogrammcode umfasst, der eingerichtet ist, den Prozessor 10 so zu steuern, dass dieser das Verfahren ausführt. In den folgenden Abschnitten werden mit Bezug zu den Figuren 2, 3 und 4 mögliche Schrittfolgen für die Ausführung des Verfahrens beschrieben.

Wie in der Figur 2 dargestellt ist, werden im Schritt S1 Augendaten in den Speicher 11 geladen.

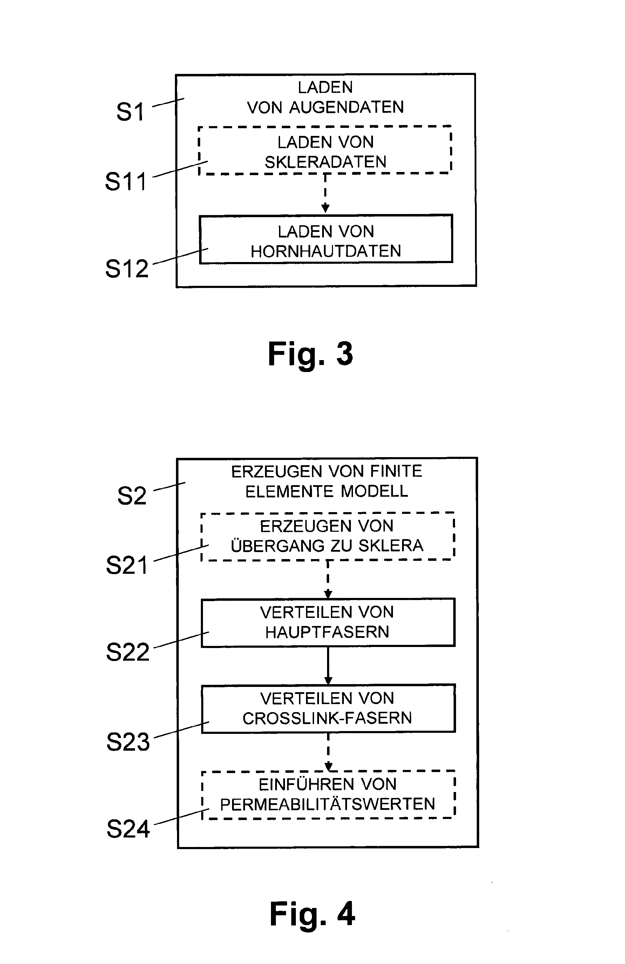

Wie in Figur 3 ersichtlich ist, werden dabei im optionalen Schritt S11 populationsbasierte Skleradaten in den Speicher 11 geladen. Die populationsbasierten Skleradaten definieren ein geometrisches Durchschnittsmodell der Sklera einschliesslich dreidimensionaler Geometrie und Abmessungen einer durchschnittlichen Sklera, auf einer statistischen Datenbasis. In einer Ausführungsvariante definieren die Skleradaten mindestens die Geometrie und die Abmessungen eines Grenzbereichs der Sklera, der an den Übergang zur Hornhaut angrenzt. Die Skleradaten werden beispielsweise von einem internetbasierten Datenquelle bezogen oder sind im Datenspeicher 11 fest gespeichert.

Im Schritt S12 werden patientenspezifische Hornhautdaten in den Speicher 11 geladen. Die patientenspezifischen Hornhautdaten definieren ein patientenspezifisches Geometriemodell der Hornhaut einschliesslich dreidimensionaler Geometrie und Abmessungen der Hornhaut eines Patienten auf einer messtechnisch erfassten Datenbasis. Die patientenspezifischen Hornhautdaten werden vorzugsweise mittels einer ophthalmologischen Messvorrichtung erfasst und im Datenspeicher 11 dem betreffenden Patienten zugeordnet gespeichert.

Wie in der Figur 2 schematisch dargestellt ist, erzeugt der Prozessor 10 im Schritt S2 ein initiales Finite Elemente Modell basierend auf den geladenen Augendaten, insbesondere ein patientenspezifisches Finite Elemente Modell der Hornhaut basierend auf den patientenspezifischen Hornhautdaten.

In einer Ausführungsvariante wird initial ein sphärisches Finite Elemente Netz erzeugt, dass mittels eines Transformationsverfahrens (Warping Method) in die patientenspezifische (durch die patientenspezifischen Hornhautdaten definierte) Geometrie der Hornhaut 34 transformiert wird, beispielsweise ein Transformationsverfahren, wie es beschrieben wird in Studer HP, Riedwyl H, Amstutz CA, Hanson VM, Büchler P, (2013); Patient-specific finite-element simulation of the human cornea: A clinical validation study on cataract surgery; Journal of Biomechanics, 46(4): 751-758.

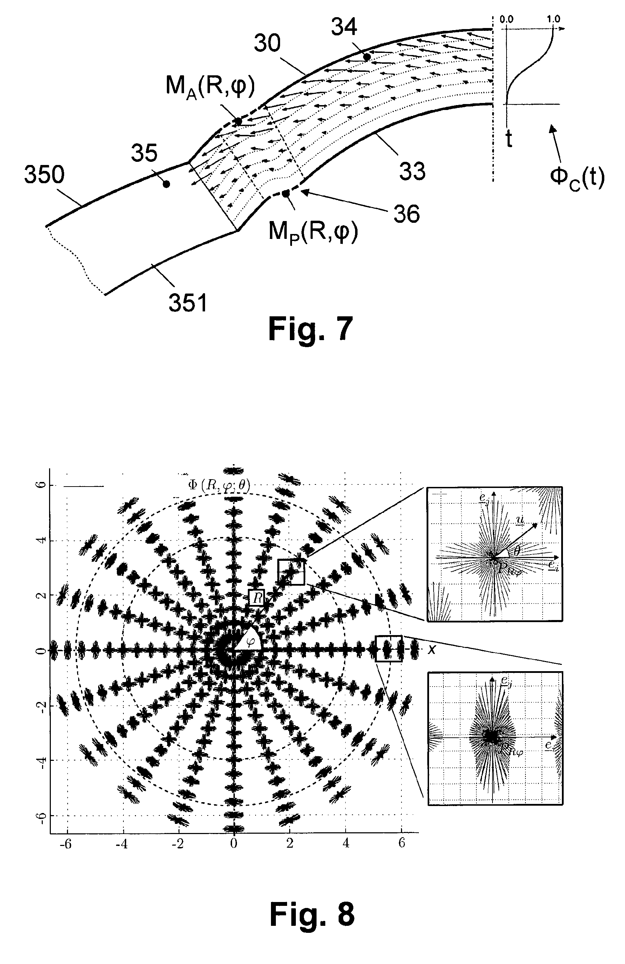

Wie in Figur 4 ersichtlich ist, wird im optionalen Schritt S21 vom Prozessor 10 ein Übergang von der patientenspezifischen Hornhaut zur populationsbasierten Sklera erzeugt. Dies ist von Vorteil weil für ein präzises Simulieren von chirurgischen Eingriffen auch ein Teil der Sklera mitsimuliert wird. Wie in der Figur 7 illustriert ist, wird ein glatter geometrischer Übergang 36 von der patientenspezifischen Hornhaut 34 zur populationsbasierten Sklera 35 erzeugt. Insbesondere werden für den Übergang 36 von der patientenspezifischen Hornhaut 34 zur populationsbasierten Sklera 35 mathematische Funktionen für geometrische Oberflächen definiert, eine Funktion $M_A(R,\varphi)$ für die vordere/äussere Übergangsoberfläche (anterior) und eine Funktion $M_P(R,\varphi)$ für die hintere/innere Übergangsoberfläche (posterior). Die vordere/äussere Übergangsoberfläche $M_A(R,\varphi)$ ist als glatte Fläche ausgebildet und erstreckt sich stetig von der vorderen/äusseren Oberfläche 30 der Hornhaut 34 zur vorderen/äusseren Oberfläche 350 der Sklera 35. Die hintere/innere Übergangsoberfläche $M_P(R,\varphi)$ ist als glatte Fläche ausgebildet und erstreckt sich stetig von der hinteren/inneren Oberfläche 33 der Hornhaut 34 zur hinteren/inneren Oberfläche 351 der Sklera 35. Abhängig von der Ausführungsvariante erfolgt die Erzeugung des Übergangs 36 auf der Grundlage des im Schritt S1 geladenen patientenspezifischen Geometriemodells der Hornhaut 34 oder auf der Grundlage des entsprechenden Finite Elemente Modells der Hornhaut 34.

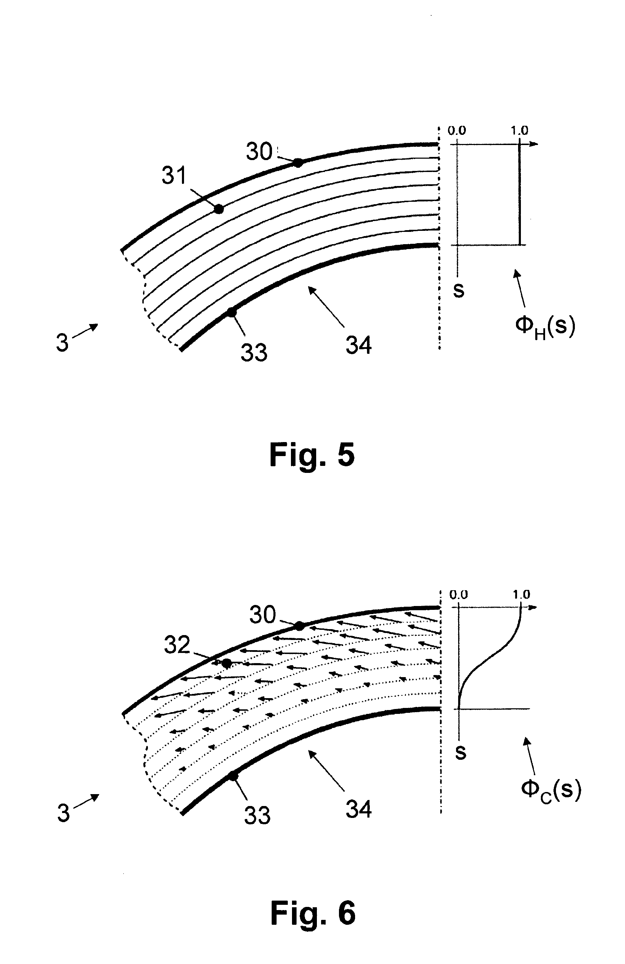

Im Schritt S22 verteilt der Prozessor 10 die, wie in Figur 5 dargestellt, parallel zur Oberfläche 30 der Hornhaut verlaufenden Hauptfasern 31 im patientenspezifischen Finite Elemente Modell der Hornhaut 34. Gegebenenfalls werden die Hauptfasern 31 entsprechend auch im Übergang 36 von der patientenspezifischen Hornhaut 34 zur populationsbasierten Sklera 35 verteilt.

Die Hauptfasern 31 werden auf Schichten oder Flächen der Hornhaut 34 verteilt, die jeweils in unterschiedlicher Tiefe, parallel zur Hornhautoberfläche 30 verlaufen. Dabei wird eine zwei-dimensionale Verteilungsfunktion $\Phi_{2D}$ verwendet (siehe Beispiel in Figur 8) um die Hauptfasern 31 jeweils innerhalb einer dieser Hornhautschichten (in-plane) zu verteilen. Diese zwei-dimensionale Verteilungsfunktion $\Phi_{2D}$ besteht dabei aus zwei Teilen:

$$\Phi_a(R, \varphi; \theta) = \cos^{2n} \theta + \sin^{2n} \theta + c_1,$$
$$\Phi_b(R, \varphi; \theta) = \sin^{2n} (\theta - \varphi) + c_2, \tag{1}$$

welche wie folgt kombiniert werden:

$$\Phi_{2D}(R, \varphi; \theta) = w \cdot \Phi_a(R, \varphi; \theta) + (1 - w) \cdot \Phi_b(R, \varphi; \theta) \tag{2}$$

Die Gewichtung $W$ zwischen den beiden Teilen hängt vom Ort eines Materialpunkts $P_{R\varphi}$ auf der Hornhautschicht ab und ist gegeben durch:

$$w = \begin{cases} 1.0 & if\ 0 < R < 4.0 \\ \frac{1}{2}\cos\left[\frac{\pi}{1.5}(R - 4.0)\right] + \frac{1}{2} & if\ 4.0 < R < 5.5 \\ 0.0 & otherwise \end{cases} \tag{3}$$

dabei ist R die Distanz des Materialpunkts $P_{R\varphi}$ zum Hornhautzentrum (0,0), $\varphi$ ist der Winkel zwischen der positiven x-Achse im Modell und der Verbindung zwischen dem Materialpunkt $P_{R\varphi}$ und dem Hornhautzentrum, und $\theta$ definiert die Faserrichtung im lokalen Koordinatensystem des Materialpunkts $P_{R\varphi}$.

Für die Verteilung (Gewichtung) der Hauptfasern 31 auf die einzelnen Hornhautschichten wird eine erste Tiefenverteilungsfunktion $\Phi_H(s)$ verwendet. Im Beispiel der Figur 5 ist die erste Tiefenverteilungsfunktion $\Phi_H(s)$ uniform (konstant), das heisst die Hauptfasern 31 werden gleichmässig über die Tiefe $s$, von der vorderen Hornhautoberfläche 30 zur hinteren Hornhautoberfläche 33, verteilt, und es hat in jeder Hornhauttiefe (in jeder Hornhautschicht) gleich viele Hauptfasern 31:

$$\Phi_H(s) = 1 \tag{4}$$

Demnach sind die Hauptfasern 31 unabhängig von der Tiefe, parallel zur Oberfläche 30 wie in Figur 8 illustriert gemäss den Gleichungen (1) bis (3) verteilt. Der Fachmann wird verstehen, dass aufgrund anderer anatomischen Gegebenheiten auch eine andere Tiefenverteilungsfunktion $\Phi_H(s)$ für die Hauptfasern 31 einsetzbar ist.

Da Teile des Übergangs 36 von der Hornhaut zur Sklera 35 Hornhautgewebe sind, wird die Faserverteilung auch im Übergang 36 vorgenommen. Die Hauptfasern 31 werden auch im Übergang 36 von der patientenspezifischen Hornhaut 34 zur populationsbasierten Sklera 35 auf die selbe Art wie in der Hornhaut 34 verteilt, innerhalb der parallelen Schichten (Flächen) mittels der zwei-dimensionalen Verteilungsfunktion $\Phi_{2D}$ und über die Tiefe $s$ gemäss der uniformen ersten Tiefenverteilungsfunktion (Gewichtungsfunktion) $\Phi_H(s)$. Die homogene Tiefenverteilung der Hauptfasern 31 ist in den Figuren 5 und 7 schematisch so dargestellt, dass die Hauptfasern 31 als parallel zur Hornhautoberfläche 30 verlaufende Schichten respektive Flächen dargestellt sind.

Im Schritt S23 verteilt der Prozessor 10 die, wie in Figur 6 dargestellt, nicht parallel zur Oberfläche 30 der Hornhaut verlaufenden, inklinierten Crosslink-Fasern 32 im patientenspezifischen Finite Elemente Modell der Hornhaut 34. Gegebenenfalls werden die Crosslink-Fasern 32 entsprechend auch im Übergang 36 von der patientenspezifischen Hornhaut 34 zur populationsbasierten Sklera 35 verteilt.

Die Crosslink-Fasern 32 sind somit eine von den Hauptfasern 31 unterschiedliche Faserfamilie, welche um ca. 15° inkliniert sind (wobei die Inklination als Abweichung zur Richtung der Hauptfasern zu verstehen ist), abwechselnd "nach innen", also der Oberfläche 33 zugeneigt, und "nach aussen", also der Oberfläche 30 zugeneigt. In einer Variante sind den Hauptfasern 31 und den Crosslink-Fasern 32 unterschiedliche Materialeigenschaften zugeordnet.

Die Crosslink-Fasern 32 werden wie die Hauptfasern 31 mittels der oben beschriebenen zwei-dimensionalen Verteilungsfunktion $\Phi_{2D}$, gemäss den Gleichungen (1) bis (3), innerhalb der parallel zur Hornhautoberfläche 30 verlaufenden Hornhautschichten respektive Flächen (in-plane) verteilt.

Für die Verteilung (Gewichtung) der Crosslink-Fasern 32 auf die einzelnen Hornhautschichten wird eine zweite Tiefenverteilungsfunktion $\Phi_C(s)$ verwendet. Im Beispiel der Figur 6 weist die zweite Tiefenverteilungsfunktion (Gewichtungsfunktion) $\Phi_C(s)$ einen mit der Tiefe $s$ abnehmenden Verlauf auf. Das heisst die Crosslink-Fasern 32 werden mit abnehmender Anzahl über die Tiefe $s$, von der vorderen Hornhautoberfläche 30 zur hinteren Hornhautoberfläche 33, verteilt. Im Gegensatz zu der Tiefenverteilung der Hauptfasern 31 werden die Crosslink-Fasern 32 somit nicht uniform, sondern mit einer spezifischen Gewichtungsfunktion über die Tiefe $s$ verteilt. Im Beispiel der Figur 6 weist die zweite Tiefenverteilungsfunktion $\Phi_C(s)$ einen sinusförmigen Verlauf auf und ist wie folgt definiert:

$$\Phi_C(s) = \begin{cases} \mathbf{1.0} & \text{if } s < \frac{1}{3} \\ \frac{1}{2}\sin\left[3\pi\left(s - \frac{1}{3}\right) + \frac{\pi}{2}\right] + 1 & \text{if } \frac{1}{3} < s < \frac{2}{3} \\ \mathbf{0.0} & \text{otherwise} \end{cases} \qquad (5)$$

Im Unterschied zu der uniformen Tiefenverteilung $\Phi_H(s)$ der Hauptfasern 31 nehmen die Crosslink-Fasern 32 mit der Tiefe $s$ in der Hornhaut 34 kontinuierlich ab. Der Fachmann wird verstehen, dass aufgrund anderer anatomischen Gegebenheiten auch eine andere über die Tiefe $s$ abnehmende Tiefenverteilungsfunktion $\Phi_C(s)$ für die Crosslink-Fasern 32 einsetzbar ist.

Da Teile des Übergangs 36 von der Hornhaut zur Sklera 35 Hornhautgewebe sind, wird die Faserverteilung und Berücksichtigung der Tiefeninhomogenität auch im Übergang 36 vorgenommen. Die Crosslink-Fasern 32 werden auch im Übergang 36 von der patientenspezifischen Hornhaut 34 zur populationsbasierten Sklera 35 auf dieselbe Art wie in der Hornhaut 34 verteilt. Innerhalb der parallel zur durch die Funktion $M_A(R,\varphi)$ definierten vorderen/äusseren Oberfläche des Übergangs 36 verlaufenden Schichten werden die Crosslink-Fasern 32 mittels der oben beschriebenen zwei-dimensionalen Verteilungsfunktion $\Phi_{2D}$ (in-plane), gemäss den Gleichungen (1) bis (3), verteilt. Die inhomogene Tiefenverteilung wird aus der Tiefenverteilung gemäss Gleichung (5) abgeleitet. Dabei gilt $t(R,\varphi) = M_A(R,\varphi) - M_P(R,\varphi)$ und somit

$$\Phi_C(t) = \begin{cases} \mathbf{1.0} & \text{if } t < \frac{1}{3} \\ \frac{1}{2}\sin\left[3\pi\left(t - \frac{1}{3}\right) + \frac{\pi}{2}\right] + 1 & \text{if } \frac{1}{3} < t < \frac{2}{3} \\ \mathbf{0.0} & \text{otherwise} \end{cases} \qquad (6)$$

Die inhomogene Tiefenverteilung der Crosslink-Fasern 32 ist in den Figuren 6 und 7 schematisch so dargestellt, dass die Crosslink-Fasern 32 als inklinierte Pfeile dargestellt sind, deren Länge ihre Gewichtung (Anzahl, Häufigkeit) symbolisiert.

Refraktiv chirurgische Behandlungen, wie zum Beispiel PhotoTherapeutic Keratectomy (PTK) und Laser Assisted Insitu Keratomileusis (LASIK), sind dafür bekannt, zu peripheren Hornhautverdickungen zu führen. Es wird angenommen, dass die Einschnitte der Behandlung zu Entspannung im Gewebe führt. Diese Entspannung wiederum führt zu Veränderungen im physiologischen Imbibitionsdruck der Hornhaut, was Flüssigkeitsverschiebungen und ein Anschwellen des Gewebes zur Folge hat. Flüssigkeitsverschiebungen durch die Gewebestruktur der Hornhaut hindurch können als poroelastisches Problem formuliert, und mittels biphasischen Finiten Elementen modelliert werden. Dabei kann sowohl der lineare, als auch der nicht-lineare Fall (im letzteren hängt die Materialpermeabilität von der Deformation ab) abgebildet werden. Im Schritt S24 führt der Prozessor im Finite Elemente Modell der Hornhaut 34 und gegebenenfalls des Übergangs zur Sklera 35 Permeabilitätswerte ein. Die Permeabilitätswerte *P(s)* charakterisieren den Flüssigkeitsaustausch im Hornhautgewebe und werden abhängig von der Tiefe *s* der Hornhaut 34 festgelegt. Da die Tiefenverteilungsfunktion für inklinierte Fasern direkt mit dem Grad der Verwachsung der Kollagenfasern und somit der Undurchlässigkeit des Gewebes zusammenhängt, kann die von der Tiefe *s* abhängige Veränderung der Permeabilität *P(s)* gemäss der nachfolgenden Gleichung (7) abhängig von der Tiefenverteilungsfunktion $\Phi_C(s)$ der Crosslink-Fasern 32 modelliert werden:

$$P(s) = 1 - \Phi_C(s). \tag{7}$$

Somit kann die Veränderung der Permeabilität in den biphasischen finiten Elementen über die Tiefe *s* der Hornhaut und damit das tiefenabhängige Anschwellen der Hornhaut modelliert werden.

Die einzelnen Elemente des im Schritt S2 erzeugten Finite Elemente Modells mit der initialen Geometrie der Hornhaut 34 und des Übergangs 36 zur Sklera 35 umfassen jeweils mehrere Integrationspunkte mit Elementwerten wie Spannung (Stress), Dehnung (Strain), Deformation, Reaktionskraft (Reaction Force), plastische Verformung, Dehnungsenergie (Strain Energy), etc. Dabei sind diese Elementwerte richtungsabhängige Werte, die an den Integrationspunkten aus den von den Fasern beigetragen Teilen berechnet werden. In einer Ausführungsvariante werden in den Elementen unterschiedliche Materialeigenschaften für die Hauptfasern 31 und die Crosslink-Fasern 32 vorgesehen und gespeichert.

Im Schritt S3 führt der Prozessor 10 die Lösungsberechnung des Finite Elemente Modells durch. Dabei wird das im Schritt S2 erzeugte Finite Elemente Modell der initialen Geometrie der Hornhaut 34 und des Übergangs 36 zur Sklera 35 (rechnerisch) dem (beispielsweise patientenspezifisch gemessenen) intraokularen Druck ausgesetzt. Dies führt im Finite Elemente Modell zu einem (errechneten) mechanischen Ungleichgewicht. In Folge wird die durch das im Finite Elemente Modell dargestellte Hornhaut verformt (aufgeblasen) und die einzelnen Elemente werden im Modell deformiert. Es entsteht also eine (errechnete) Dehnung in den Elementen des Modells. Die Dehnung wird mittels einer definierten Materialfunktion in Verbindung gebracht, welche bestimmt, wie viel Spannung aufgrund einer errechneten Dehnung entsteht. Tatsächlich fliessen die Fasern und Verteilungsfunktionen in diese Materialfunktion ein, welche an jedem Integrationspunkt berechnet wird. Die Materialfunktion stellt Dehnung und Energie (stress) in Zusammenhang. Die Materialfunktion ist definiert durch

$$\Psi = U + \overline{\Psi}_m[C_{10}] + \frac{1}{\pi}\int\Phi\left(\overline{\Psi}_{f1}[\gamma_m, \mu_m] + \overline{\Psi}_{f2}[\gamma_k, \mu_k]\right)d\theta \tag{8}$$

wobei $\psi$ die Energie, $\overline{\psi}_{f1}$ die Hauptfasern 31, $\overline{\psi}_{f2}$ die Crosslink-Fasern 32 und $\Phi$ die Verteilungsfunktionen sind. $\overline{\psi}_m$ ist ein Gewebeteil, der nicht aus Fasern besteht, und **U** ist eine Straffunktion für Volumenänderungen (dies führt zur Inkompressibilität des Gewebes). Die errechnete Spannung wird dann wiederum im Element gespeichert. Diese Abfolge Deformation -> Dehnung -> Spannung wird im Schritt S3 iterativ so lange wiederholt, bis die Dehnungskonfiguration erreicht ist, bei welcher mit den errechneten Spannungen der angelegte intraokulare Druck ausgeglichen wird. Wenn für jedes Element der Deformationszustand respektive die Dehnungskonfiguration für den Ausgleich des intraokularen Drucks gefunden ist, liegt ein errechnetes Gleichgewicht aus Spannungen in den Elementen, welche aus der Deformation und Dehnung resultieren, und dem intraokularen Druck, also eine Lösung des Finite Elemente Modells vor.

Im Schritt S4 stellt der Prozessor 10, basierend auf dem erzeugten und sich mit dem intraokularen Druck im Gleichgewicht befindenden Finite Elemente Modell, auf der Anzeige 12 ein dreidimensionales Modell der Hornhaut 34 grafisch dar. Die grafische Darstellung des Hornhautmodells kann von einem Benutzer der Vorrichtung 1 vorzugsweise über Eingabe von Benutzerbefehlen (z.B. via Maus, Tastatur und/oder Touchscreen) verändert werden, beispielsweise wie bei einem CAD-System hinsichtlich Orientierung durch freies Drehen im Raum und/oder hinsichtlich Grösse respektive Auflösung durch eine Zoomfunktion.

Im Schritt S5 nimmt der Prozessor 10 Schnittdaten entgegen, beispielsweise gemäss Benutzerbefehlen von einem Benutzer der Vorrichtung 1 und/oder aus einer selektierten Datei mit gespeicherten Schnittdaten. Die Benutzerbefehle zur Definition von Schnitten respektive entsprechenden Schnittdaten können vom Benutzer beispielsweise mittels einer Computermaus, einer Tastatur und/oder über einen Touchscreen im dargestellten Modell eingegeben werden. Die Schnittdaten definieren die dreidimensionale Geometrie, die Abmessungen und die Position von einem oder mehreren Schnitten in der Hornhaut 34 und/oder in Bereichen von der Hornhaut 34 durch den Übergang 36 in die angrenzende Sklera 35. Dabei können die Schnitte auch im Innern des Augengewebes positioniert sein, mit oder ohne Durchtrennung der vorderen/äusseren Oberfläche 30 der Hornhaut 35 respektive der Übergangsoberfläche $M_A(R,\varphi)$ des Übergangs 36 und der vorderen/äusseren Oberfläche 350 der Sklera 35.

Im Schritt S6 werden die Schnittdaten vom Prozessor 10 auf das Finite Elemente Modell der Hornhaut 34 und des Übergangs 36 angewendet. Das heisst der Prozessor 10 bestimmt die durch die Schnittdaten betroffenen Elemente im Modell, die durch einen gemäss den Schnittdaten durchgeführten Schnitt voneinander getrennt werden.

Im Schritt S3 führt der Prozessor 10 erneut die Lösungsberechnung des Finite Elemente Modells, wie oben beschrieben, für das durch die Schnittdaten veränderte Finite Elemente Modell durch. Im Schritt S4 erzeugt der Prozessor 10 auf der Anzeige 12 eine grafische Darstellung des durch den Schnitt veränderten Hornhautmodells basierend auf dem veränderten und sich wieder mit dem intraokularen Druck im Gleichgewicht befindenden Finite Elemente Modell.

Das Finite Elemente Modell ermöglicht somit die Modellierung der Hornhaut 34 und die Simulation von Gewebeschnitten in der Hornhaut 35. Dabei ist der Iterationsgrad der Schritte S3 -> S4 -> S5 -> S6 -> S3, respektive die Länge von Schnitten pro Iterationsschritt, beispielsweise vom Benutzer einstellbar.

Abschliessend soll angeführt werden, dass in der Beschreibung zwar die Ausführung von Schritten in einer bestimmten Reihenfolge dargestellt wurde, dass der Fachmann jedoch verstehen wird, dass die Reihenfolge von mindestens gewissen Schritten geändert werden kann, ohne dabei vom Schutzgegenstand abzuweichen.

**Patentansprüche**

1. Computerisierte Vorrichtung (1) zur Modellierung einer Hornhaut (34) für die Simulation von Gewebeschnitten in der Hornhaut (34), umfassend einen Prozessor (10), der programmiert ist, ein patientenspezifisches Finite Elemente Modell der Hornhaut (34) zu erzeugen, **dadurch gekennzeichnet, dass** der Prozessor (10) überdies programmiert ist, eine erste Gruppe von Gewebefasern, mit parallel zur äusseren Oberfläche (30) der Hornhaut (34) verlaufenden Hauptfasern (31), gemäss einer ersten Verteilungsfunktion im Finite Elemente Modell zu verteilen, und eine zweite Gruppe von Gewebefasern, mit inklinierten, nicht parallel zur Oberfläche der Hornhaut (34) verlaufenden Crosslink-Fasern (32), gemäss einer zweiten Verteilungsfunktion im Finite Elemente Modell zu verteilen, wobei die zweite Verteilungsfunktion die Crosslink-Fasern (32) mit einer nicht uniformen Gewichtungsfunktion ($\Phi_C(s)$) über die Tiefe (s) der Hornhaut (34), von der äusseren Oberfläche (30) der Hornhaut (34) zur inneren Oberfläche der Hornhaut (34), verteilt.

2. Vorrichtung (1) nach Anspruch 1, **dadurch gekennzeichnet, dass** der Prozessor (10) programmiert ist, die Crosslink-Fasern (32) mit einer abnehmenden Gewichtungsfunktion ($\Phi_C(s)$) über die Tiefe (s) der Hornhaut (34) zu verteilen.

3. Vorrichtung (1) nach einem der Ansprüche 1 oder 2, **dadurch gekennzeichnet, dass** der Prozessor (10) programmiert ist, im Finite Elemente Modell Permeabilitätswerte für Flüssigkeitsaustausch im Hornhautgewebe, einzuführen, welche von der Tiefe (s) der Hornhaut (34) abhängig sind.

4. Vorrichtung (1) nach einem der Ansprüche 2 oder 3, **dadurch gekennzeichnet, dass** der Prozessor (10) programmiert ist, im Finite Elemente Modell Permeabilitätswerte für Flüssigkeitsaustausch im Hornhautgewebe, einzuführen, welche von der Gewichtungsfunktion ($\Phi_C(s)$) abhängig sind, mit der die Crosslink-Fasern (32) über die Tiefe (s) der Hornhaut (34) verteilt sind.

5. Vorrichtung (1) nach einem der Ansprüche 1 bis 4, **dadurch gekennzeichnet, dass** der Prozessor (10) programmiert ist, einen Übergang (36) vom patientenspezifischen Finite Elemente Modell der Hornhaut (34) auf ein populationsbasiertes Skleramodell (35) zu erzeugen, und die Crosslink-Fasern (32) im Übergang (36) mit einer abnehmenden Gewichtungsfunktion ($\Phi_C(t)$) über die Tiefe (t) des Übergangs (36) zu verteilen, von einer äusseren Übergangsoberfläche ($M_A(R,\varphi)$, die sich von der äusseren Oberfläche (30) der Hornhaut (34) zur äusseren Oberfläche (350) der Sklera (35) erstreckt, zu einer inneren Übergangsoberfläche ($M_P(R,\varphi)$), die sich von der inneren Oberfläche (33) der Hornhaut (34) zur inneren Oberfläche (351) der Sklera (35) erstreckt.

6. Vorrichtung (1) nach einem der Ansprüche 1 bis 5, **dadurch gekennzeichnet, dass** der Prozessor (10) programmiert ist, die Crosslink-Fasern (32) im Finite Elemente Modell auf mehrere parallel zur äusseren Oberfläche der Hornhaut (34) verlaufenden Schichten zu verteilen, und die Crosslink-Fasern (32) in den Schichten jeweils mit einer zweidimensionalen Verteilungsfunktion ($\Phi_{2D}$) zu verteilen.

7. Vorrichtung (1) nach einem der Ansprüche 1 bis 6, **dadurch gekennzeichnet, dass** der Prozessor (10) programmiert ist, im Finite Elemente Modell unterschiedliche Materialeigenschaften für die erste Gruppe von Gewebefasern und die zweite Gruppe von Gewebefasern zu speichern.

8. Vorrichtung (1) nach einem der Ansprüche 1 bis 7, **dadurch gekennzeichnet, dass** der Prozessor (10) programmiert ist, die Hauptfasern (31) im Finite Elemente Modell gleichmässig auf mehrere parallel zur äusseren Oberfläche (30) der Hornhaut (34) verlaufenden Flächen zu verteilen und auf den Flächen jeweils mit einer zweidimensionalen Verteilungsfunktion ($\Phi_{2D}$) zu verteilen.

9. Computerimplementiertes Verfahren zur Modellierung einer Hornhaut (34) für die Simulation von Gewebeschnitten in der Hornhaut (34), welches Verfahren eine Ausführung folgender Schritte durch einen Prozessor (10) umfasst:

Erzeugen (S2) eines patientenspezifischen Finite Elemente Modells der Hornhaut (34),
Verteilen (S22) im Finite Elemente Modell einer ersten Gruppe von Gewebefasern, mit parallel zur Oberfläche der Hornhaut (34) verlaufenden Hauptfasern (31), gemäss einer ersten Verteilungsfunktion, und Verteilen (S23) im Finite Elemente Modell einer zweiten Gruppe von Gewebefasern, mit inklinierten, nicht parallel zur Oberfläche der Hornhaut (34) verlaufenden Crosslink-Fasern (32), gemäss einer zweiten Verteilungsfunktion, wobei die zweite Verteilungsfunktion die Crosslink-Fasern (32) mit einer nicht uniformen Gewichtungsfunktion ($\Phi_C(s)$) über die Tiefe (s) der Hornhaut (34), von der äusseren Oberfläche (30) der Hornhaut (34) zur inneren Oberfläche (33) der Hornhaut (34), verteilt.

10. Verfahren nach Anspruch 9, **gekennzeichnet durch** Verteilen (S23) der Crosslink-Fasern (32) **durch** den Prozessor (10) mit einer abnehmenden Gewichtungsfunktion $\Phi_C(s)$ über die Tiefe (s) der Hornhaut (34).

11. Verfahren nach einem der Ansprüche 9 oder 10, **gekennzeichnet durch** Einführen (S24) ins Finite Elemente Modell **durch** den Prozessor (10) von Permeabilitätswerten für Flüssigkeitsaustausch im Hornhautgewebe, welche von der Tiefe (s) der Hornhaut (34) abhängig sind.

12. Verfahren nach einem der Ansprüche 10 oder 11, **gekennzeichnet durch** Einführen (S24) ins Finite Elemente Modell **durch** den Prozessor (10) von Permeabilitätswerten für Flüssigkeitsaustausch im Hornhautgewebe, welche von der Gewichtungsfunktion $\Phi_C(s)$ abhängig sind, mit der die Crosslink-Fasern (32) über die Tiefe (s) der Hornhaut (34) verteilt sind.

13. Verfahren nach einem der Ansprüche 9 bis 12, **gekennzeichnet durch** Erzeugen (S21) **durch** den Prozessor (10) eines Übergangs (36) vom patientenspezifischen Finite Elemente Modell der Hornhaut (34) auf ein populationsbasiertes Skleramodell (35), und Verteilen **durch** den Prozessor (10) der Crosslink-Fasern (32) im Übergang (36) mit einer abnehmenden Gewichtungsfunktion $\Phi_C(t)$ über die Tiefe (t) des Übergangs (36), von einer äusseren Übergangsoberfläche **($M_A(R,\Phi)$)**, die sich von der äusseren Oberfläche (30) der Hornhaut (34) zur äusseren Oberfläche (350) der Sklera (35) erstreckt, zu einer inneren Übergangsoberfläche $M_P(R,\varphi)$**,** die sich von der inneren Oberfläche (33) der Hornhaut (34) zur inneren Oberfläche (351) der Sklera (35) erstreckt.

14. Verfahren nach einem der Ansprüche 9 bis 13, **gekennzeichnet durch** Verteilen (S23) **durch** den Prozessor (10) der Crosslink-Fasern (32) im Finite Elemente Modell auf mehrere parallel zur äusseren Oberfläche der Hornhaut (34) verlaufenden Schichten, und **durch** Verteilen (S23) **durch** den Prozessor (10) der Crosslink-Fasern (32) in den Schichten jeweils mit einer zweidimensionalen Verteilungsfunktion.

15. Computerprogrammprodukt umfassend ein nicht-transientes computerlesbares Medium mit darauf gespeichertem Computerprogrammcode, welcher eingerichtet ist, einen Prozessor (10) derart zu steuern, dass der Prozessor (10) das Verfahren nach einem der Ansprüche 9 bis 14 ausführt.

1 ↗

10    11    2

**Fig. 1**

S1 — LADEN
VON AUGENDATEN

S2 — ERZEUGEN VON FINITE
ELEMENTE MODELL

S3 — LÖSUNGSBERECHNUNG DES
FINITE ELEMENTE MODELLS

S4 — DARSTELLEN VON FINITE
ELEMENTE MODELL

S5 — ERFASSEN VON
SCHNITTDATEN

S6

**Fig. 2**

S1 — LADEN
VON AUGENDATEN

S11 — LADEN VON
SKLERADATEN

S12 — LADEN VON
HORNHAUTDATEN

## Fig. 3

S2 — ERZEUGEN VON FINITE
ELEMENTE MODELL

S21 — ERZEUGEN VON
ÜBERGANG ZU SKLERA

S22 — VERTEILEN VON
HAUPTFASERN

S23 — VERTEILEN VON
CROSSLINK-FASERN

S24 — EINFÜHREN VON
PERMEABILITÄTSWERTEN

## Fig. 4

**Fig. 5**

**Fig. 6**

**Fig. 7**

**Fig. 8**

Europäisches Patentamt
European Patent Office
Office européen des brevets

**EUROPÄISCHER RECHERCHENBERICHT**

Nummer der Anmeldung

EP 13 00 3481

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | HARALD P. STUDER ET AL: "Patient-specific finite-element simulation of the human cornea: A clinical validation study on cataract surgery", JOURNAL OF BIOMECHANICS, Bd. 46, Nr. 4, 1. Februar 2013 (2013-02-01), Seiten 751-758, XP055089802, ISSN: 0021-9290, DOI: 10.1016/j.jbiomech.2012.11.018 * das ganze Dokument * ----- | 1-15 | INV. G06F19/00 A61B19/00 G06F17/50 |
| A,D | STUDER H ET AL: "Biomechanical model of human cornea based on stromal microstructure", JOURNAL OF BIOMECHANICS, PERGAMON PRESS, NEW YORK, NY, US, Bd. 43, Nr. 5, 22. März 2010 (2010-03-22), Seiten 836-842, XP026937973, ISSN: 0021-9290, DOI: 10.1016/J.JBIOMECH.2009.11.021 [gefunden am 2010-03-03] * das ganze Dokument * ----- | 1-15 | |
| A | AGHAMOHAMMADZADEH ET AL: "X-Ray Scattering Used to Map the Preferred Collagen Orientation in the Human Cornea and Limbus", STRUCTURE, CURRENT BIOLOGY LTD., PHILADELPHIA, PA, US, Bd. 12, Nr. 2, 12. Februar 2004 (2004-02-12), Seiten 249-256, XP022553540, ISSN: 0969-2126 * das ganze Dokument * ----- -/-- | 1-15 | RECHERCHIERTE SACHGEBIETE (IPC) G06F A61B |

Der vorliegende Recherchenbericht wurde für alle Patentansprüche erstellt

| Recherchenort | Abschlußdatum der Recherche | Prüfer |
|---|---|---|
| Den Haag | 16. Dezember 2013 | Schechner-Resom, M |

EPO FORM 1503 03.82 (P04C03)

**Europäisches Patentamt**
**European Patent Office**
**Office européen des brevets**

## EUROPÄISCHER RECHERCHENBERICHT

Nummer der Anmeldung

EP 13 00 3481

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | ROY ABHIJIT SINHA ET AL: "Patient-specific modeling of corneal refractive surgery outcomes and inverse estimation of elastic property changes", JOURNAL OF BIOMECHANICAL ENGINEERING, A S M E INTERNATIONAL, US, Bd. 133, Nr. 1, 1. Januar 2011 (2011-01-01), Seiten 11002-1, XP008166253, ISSN: 1528-8951, DOI: 10.1115/1.4002934 [gefunden am 2010-12-22] * das ganze Dokument * ----- | 1-15 | |
| A,D | US 2009/318907 A1 (BILLE JOSEF F [DE] ET AL) 24. Dezember 2009 (2009-12-24) * das ganze Dokument * ----- | 1-15 | |
| A | PANDOLFI ANNA ET AL: "Three-dimensional modeling and computational analysis of the human cornea considering distributed collagen fibril orientations", JOURNAL OF BIOMECHANICAL ENGINEERING, NEW YORK, NY, US, Bd. 130, Nr. 6, 1. Dezember 2008 (2008-12-01), Seiten 61006-1, XP008166090, ISSN: 0148-0731, DOI: 10.1115/1.2982251 [gefunden am 2008-10-09] * das ganze Dokument * ----- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Den Haag | 16. Dezember 2013 | Schechner-Resom, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 13 00 3481

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-12-2013

| Im Recherchenbericht angeführtes Patentdokument | Datum der Veröffentlichung | Mitglied(er) der Patentfamilie | Datum der Veröffentlichung |
|---|---|---|---|
| US 2009318907 A1 | 24-12-2009 | US 2009318907 A1<br>WO 2009153631 A2 | 24-12-2009<br>23-12-2009 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 0207660 A
- WO 9418636 A
- US 2009318907 A
- US 2009187386 A

### In der Beschreibung aufgeführte Nicht-Patentliteratur

- **QUANTOCK AJ ; BOOTE C ; YOUNT RD ; HAYES S ; TANIOKA H ; KAWASAKI S ; OHTA N ; LIDA T ; YAGI N ; KINOSHITA S.** Small-angle fibre diffraction studies of corneal matrix structure: a depth-profiled investigation of the human eye-bank cornea. *Journal of Applied Crystallography,* 2007, vol. 40, 335-340
- **PALKA BP ; TANIOKA H ; SOTOZONO C ; YAGI N ; BOOTE C ; YOUNG RD ; MEEK KM ; QUANTOCK AJ.** Reduced collagen interfibrillar spacing in macular corneal dystrophy occurs predominantly in deep stromal layers. *Investigative Ophthalmology & Visual Science,* 2008, vol. 49
- **KAMMA-LORGER CS ; BOOTE C ; YOUNG RD ; HAYES S ; QUANTOCK AJ ; MEEK KM.** Depth profile study of molecular collagen structure in normal human cornea. *Acta Ophthalmologica,* 2008, vol. 86, 243
- **MEEK KM ; BOOTE C.** The use of X-ray scattering techniques to quantify the orientation and distribution of collagen in the corneal stroma. *Progress in Retinal and Eye Research,* 2009, vol. 28, 369-392
- **KAMMA-LORGER CS ; BOOTE C ; HAYES S ; MOGER J ; BURGHAMMER M ; KNUPP C ; QUANTOCK AJ ; SORENSEN T ; DI COLA E ; WHITE N.** Collagen and mature elastic fibre organization as a function of depth in the human cornea and limbus. *Journal of Structural Biology,* 2010, vol. 169, 424-430
- **PETSCHE SJ ; CHERNAYAK D ; MARTIZ J ; LEVENSTON ME ; PINSKY PM.** Depth-Dependent Transverse Shear Properties of the Human Corneal Stroma. *Investigative Ophthalmology & Visual Science,* 2012, vol. 53 (2), 873-80
- **BRYANT M ; MCDONNELL P.** Constitutive laws for biomechanical modelling of refractive surgery. *Journal of Biomechanical Engineering,* 1996, vol. 118 (4), 473-481
- **PINSKY PM ; VAN DER HEIDE D ; CHERNYAK D.** Computational modelling of mechanical anisotropy in the cornea and sclera. *Journal of Cataract and Refractive Surgery,* 2005, vol. 31 (1), 136-145
- **ALASTRUE V ; CALVO B ; PENA E ; DOBLARE M.** Biomechanical Modelling of Refractive Surgery. *Journal of Biomechanical Engineering,* 2006, vol. 128, 150
- **LANCHARES E ; CALVO B ; CRISTOBAL J ; DOBLARE M.** Finite element simulation of arcuates for astigmatism correction. *Journal of Biomechanics,* 2008, vol. 41, 797-805
- **PANDOLFI A ; MANGANIELLO F.** A model for the human cornea: constitutive formulation and numerical analysis. *Biomechanics and Modelling in Mechanobiology,* 2006, vol. 5 (4), 237-246
- **PANDOLFI A ; HOLZAPFEL G.** Three-dimensional modelling and computational analysis of the human cornea considering distributed collagen fibril orientations. *Journal of Biomechanical Engineering,* 2008, vol. 130 (6
- **PANDOLFI A ; FOTIA G ; MANGANIELLO F.** Finite element simulation of laser refractive corneal surgery. *Engineering with Computers,* 2009, vol. 25, 15-24
- **STUDER HP ; LARREA X ; RIEDWYL H ; BÜCHLER P.** Biomechanical model of human cornea based on stromal microstructure. *Journal of Biomechanics,* 2010, vol. 43, 836-842
- **PETSCHE SJ ; PINSKY PM.** The role of 3-D collagen organization in stromal elasticity: a model based on X-ray diffraction data and second harmonic-generated images. *Biomechanics and Modelling in Mechanobiology,* 2013
- **STUDER HP ; RIEDWYL H ; AMSTUTZ CA ; HANSON VM ; BÜCHLER P.** Patient-specific finite-element simulation of the human cornea: A clinical validation study on cataract surgery. *Journal of Biomechanics,* 2013, vol. 46 (4), 751-758